# EUROPEAN PATENT SPECIFICATION

(11) **EP 4 248 940 B1**
(45) Date of publication and mention of the grant of the patent: **07.05.2025**
(21) Application number: 23161260.7
(22) Date of filing: 10.03.2023
(51) Int. Cl.: A61K 8/31, A61K 8/36, A61K 8/362, A61K 8/365, A61K 8/37, A61Q 5/02, A61Q 5/12

(54) **COMPOSITION FOR ENHANCING KERATIN FIBERS**
ZUSAMMENSETZUNG ZUR VERSTÄRKUNG VON KERATINFASERN
COMPOSITION POUR AMÉLIORER DES FIBRES DE KÉRATINE

(30) Priority: 25.03.2022 EP 22164521
(43) Date of publication of application: 27.09.2023
(73) Proprietor: Wella Germany GmbH, 64295 Darmstadt (DE)
(72) Inventor: SPRINGOB, Christian, Darmstadt (DE); RIEMANN, Ingo, Darmstadt (DE)
(74) Representative: Zimmermann & Partner Patentanwälte mbB

(56) References cited:
- EP-A1- 1 570 832
- WO-A1-2004/047777
- DATABASE GNPD [online] MINTEL; 8 October 2019 (2019-10-08), ANONYMOUS: "3 Bond Retention Mask", XP055966817, retrieved from https://www.gnpd.com/sinatra/recordpage/6908743/ Database accession no. 6908743
- DATABASE GNPD [online] MINTEL; 21 July 2021 (2021-07-21), ANONYMOUS: "Treatment", XP055966884, retrieved from https://www.gnpd.com/sinatra/recordpage/8876993/ Database accession no. 8876993
- DATABASE GNPD [online] MINTEL; 24 October 2014 (2014-10-24), ANONYMOUS: "Botanical Oil Treatment Mask", XP055966875, retrieved from https://www.gnpd.com/sinatra/recordpage/2746043/ Database accession no. 2746043
- DATABASE GNPD [online] MINTEL; 11 May 2017 (2017-05-11), ANONYMOUS: "P3 Mask", XP093068940, retrieved from https://www.gnpd.com/sinatra/recordpage/4818165/ Database accession no. 4818165

## Description

### Technical Field

The presently claimed invention relates to a hair treatment composition comprising components providing rehabilitation of damaged keratin fibers, and a method for application of the composition.

### Background

Keratin fibers and human hair are damaged by daily routines such as brushing and combing, external influences like sun light and pollution and oxidative and/or reductive treatments like hair coloration, bleaching or perming. The damage caused by these activities includes removal of the F-layer fatty acids, breakage of disulfide links of keratin protein and erosion of the keratin protein materials at the surfaces of the hair fiber cuticles. The damage tends to roughen the hair fiber surfaces much like a rasp roughens wood. The roughened hair strands lack smoothness, tend to misbehave and are unruly. Hair care goals have involved attempts to reverse these tendencies.

One such attempt has involved hair care and repair agents such as hair cortex repair formulations and associated methods. It is thought that damage to the hair cortex can result in unravelling cortex protein bundles and can lead to the unruliness and roughness. One possible answer has involved use of crosslinking of dimaleate type of molecules to re-bond cleaved disulfide links in hair cortex. See International Patent Application WO 2015/017768 A1. Another possible answer has involved a method for restructuring of hair fiber cortex with cysteine and at least one dicarboxylic acid. See WO 2005/115314 A1.

EP 1 570 832 A1 and
WO 2004/047777 A1 disclose a hair cosmetic composition comprising malic acid and lactic acid.

Frizz control is among the ultimate goals of hair treatment compositions. There is a need for high performance frizz control products, particularly, but not exclusively in "leave-in" formulations that are acceptable to the consumer. Such a formulation does ideally provide good application feel (without heaviness, stiffness or greasiness), a good ability to be smoothly distributed and spread on the hair and, when dry, such a formulation does ideally leave the hair manageable, and feeling and looking natural. A further need is the improvement of the integrity and the healthiness of hair and deliver shine, suppleness, a smooth hair feel, and good disentangling properties.

Accordingly, it was an object of the presently claimed invention to provide a hair treatment composition that shows improved conditioning properties such as improved wet and dry combability, improved wet feel, improved anti-frizz, improved hair shine and improved dry feel while preventing the hair from appearing greasy and uncleaned.

### Summary

The object was solved by the provision of a hair treatment composition comprising at least one C3-C13 saturated or unsaturated dicarboxylic acid, at least one C10-C30 saturated or unsaturated monocarboxylic acid, at least one C10-C40 saturated or unsaturated hydrocarbon, and at least one fatty acid ester of a C10-C30 saturated or unsaturated monocarboxylic acid and a C3-C6 mono-ol, diol or triol in specific amounts. It was found that use of at least one fatty acid ester of a C10-C30 saturated or unsaturated monocarboxylic acid and a C3-C6 mono-ol, diol or triol in hair treatment compositions further improved conditioning properties such as wet and dry combability, wet feel, anti-frizz, hair shine and dry feel without imparting excessive heaviness or an oily or greasy look to the hair.

Hence, in one aspect, the presently claimed invention is directed to a hair treatment composition comprising:
a) at least one C3-C13 saturated or unsaturated dicarboxylic acid which is unsubstituted or substituted with 1, 2, 3 or 4 hydroxyl groups at a weight percentage concentration of from 0.05 wt.% to 15.0 wt. %;
b) at least one C10-C30 saturated or unsaturated monocarboxylic acid at a weight percentage concentration of 0.05 wt.% to 1.0 wt.%;
c) at least one C10-C50 saturated or unsaturated hydrocarbon at a weight percentage concentration of 0.01 wt.% to 10.0 wt.%;
d) at least one fatty acid ester of a C10-C30 saturated or unsaturated monocarboxylic acid and a C3-C6 mono-ol, diol or triol at a weight percentage concentration of 0.1 wt.% to 5.0 wt.%;
e) at least one cosmetically acceptable aqueous or aqueous-organic medium;
wherein the components a) and b) are present in the form of a free acid or a cosmetically acceptable salt thereof and all weight percentages are relative to the total weight of the composition.

The presently claimed invention is directed to a hair treatment composition and corresponding method for treatment of the cuticle of keratin fibers, specifically cuticles of anagenic human hair damaged by daily routine and chemical treatment. Embodiments of the hair treatment composition deliver components to protect and repair the damaged cuticles of keratin fibers, such as anagenic hair strands of humans. The compositional components enable repair of broken keratin protein inner connections, smoothing of hair strand roughness, lubrication of hair strands and melding of split strand ends. The compositional components deliver hair strand treatment without causing heavy weighting of hair strands, oiliness, and avoid use of animal and fish components. The compositional components deliver "green technology" to the treatment and conditioning of anagenic hair.

The hair treatment composition may comprise a stand-alone hair conditioner formulation such as a rinse-off or leave on conditioner or may comprise a conditioning feature of a sulfate shampoo or sulfate-free shampoo. The components of the hair treatment composition are green-oriented in that they may be derived from plant material and/or may be synthetic and/or semi-synthetic derivatives thereof.

Additional components for incorporation into the hair treatment composition include one or more saturated or unsaturated fatty C20-C30 alcohols; one or more cationic surfactants such as, but not limited to, quaternary ammonium compounds formed of one or more saturated and/or unsaturated fatty C20 -C30 amines quaternized with one to three C1-C3 alkyl groups.

Further additives include liquid silicones, antioxidants, preservatives, anti-microbials, UV protectants, fragrance, viscosity control agents, surfactants, humectants, emollients, hair strand thickeners for providing strand body such as, but not limited to, silicas, diatomaceous earth, clays such as kaolin and smectite phyllosilicates and color and/or shine agents.

Use embodiments of the hair treatment composition according to the invention comprise a sulfate shampoo, a non-sulfate shampoo, a non-ionic surfactant hair cleaning liquid, a stand-alone conditioner, a mask, a leave on treatment, a rinse off conditioner, a hair rinse, a hair liquid, a hair oil, a hair pomade, a hair foam, a hair spray, a hair tonic and/or hair lotion, all with the hair treatment composition as at least a part of the use embodiment.

A further aspect of the presently claimed invention is directed to a method for applying the hair treatment composition and/or any of its use embodiments to hair, preferably anagenic hair.

### Detailed description

The following detailed description is merely exemplary in nature and is not intended to limit the presently claimed invention or the application and uses of the presently claimed invention. Furthermore, there is no intention to be bound by any theory presented in the preceding technical field, background, summary or the following detailed description.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

Furthermore, the terms "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the subject matter described herein are capable of operation in other sequences than described or illustrated herein. In case the terms "(A)", "(B)" and "(C)" or "(a)", "(b)", "(c)", "(d)", "(i)", "(ii)" etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

The term "about", when used in relation to a weight ratio or a weight percentage, means that a specific value should be understood as meaning a range of ± 10 %. For example, the weight proportion of a compound of about 1 %, means a weight proportion ranging from 0.9 to 1.1 %. For example, the weight proportion of a compound of about 2.5 %, means a weight proportion ranging from 2.25 to 2.75 %.

In the following passages, different aspects of the subject matter are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "preferred embodiment" means that a particular feature, structure, or characteristic described in connection with the embodiment is included in at least one embodiment of the presently claimed invention. Thus, appearances of the phrases "in one embodiment" or "in a preferred embodiment" in various places throughout this specification are not necessarily all referring to the same embodiment but may refer to different embodiments of the presently claimed invention. Furthermore, the features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the subject matter, and form different embodiments, as would be understood by those in the art. For example, in the appended claims, any of the claimed embodiments can be used in any combination.

Furthermore, the ranges defined throughout the specification include the end values as well, i.e., a range of 1 to 10 implies that both 1 and 10 are included in the range. For the avoidance of doubt, the applicant shall be entitled to any equivalents according to applicable law.

For the purposes of the presently claimed invention, '% by weight' or 'wt.% 'as used in the presently claimed invention is with respect to the total weight of the bleach powder. Further, the sum of wt.% of all the compounds, as described herein, in the respective components adds up to 100 wt.%.

The measurement techniques described hereinabove and herein are well known to a person skilled in the art and therefore do not limit the presently claimed invention.

By "user" or "customer" it is meant the person preparing the hair treatment composition. The user is for example a professional hair stylist working in a salon and is different from the subject on whose hair the composition is applied, or the user is identical to the person on whose hair the composition is applied. The mixing can take place in a bowl or within a bottle or even through a mixer placed between the stored individual compositions and the point at which it is dispensed to be used. While two component bleach powders are the most commonly found in the market, there are also others which use three or more components.

The term "anagenic hair" as used herein means hair strands that are in direct connection with a hair follicle which is in either the anagen or telogen state. Anagenic hair is present in one of these states on a scalp of a person, a human, and its follicle is appurtenant to a sebum gland that substantially continuously secretes sebum and long chain fatty acids onto the surfaces of the hair shaft. As the hair grows from the follicle, it carries with it coatings of secreted sebum and long chain fatty acids. The fatty acid coating is known as the f layer and is tightly entwined with the keratin protein at the hair shaft surface. Hair cut from a living person is no longer anagenic hair.

Keratin fibers means hair strands on the scalp of a person, preferably anagenic hair. Keratin fibers, aka hair strands, are comprised of cells forming an inner cortex as the core and a cuticle as the outer covering surrounding the cuticle. The hair strand chemical components include proteins, poly-nucleic acids, amino acids, minerals, melanin, lipids including fatty acids, fatty alcohols, triglycerides, phospholipids, cholesterol, and squalene. These components are configured as cells, fibrils, connective tissue, extracellular links, pigments, keratin and associated structures. The outer layer of the cuticle is configured as a sheath of overlapping flat cells having a structure like the bark of a palm tree or scales of a fish. Like the scales of a fish, the surface of the cuticle is smooth in the direction from the root to the tip and rough in the direction from the tip to the root.

The below materials are being used in the embodiments of the presently claimed invention. The presently claimed invention is described with non-limiting embodiments. The listed ingredients are the preferred features as well as other preferred but non-limiting features of the presently claimed invention.

The measurement techniques described hereinabove and herein are well known to a person skilled in the art and therefore do not limit the presently claimed invention.

The presently claimed invention is directed to a hair treatment composition that delivers components and substances for replenishment of, and addition to, damaged anagenic hair. The hair treatment composition may be incorporated as a facet of another cosmetic composition such as a shampoo or may formulated as a stand-alone composition such as, but not limited to, a conditioner composition.

Anagenic hair can be damaged by wear and degradation caused by perming, redox treatment, shampooing, brushing, combing and/or rubbing such as with towels, hats, caps and the like. These activities performed on anagenic hair strands cause the strands to be thinned, broken, fragmented, splintered and otherwise damaged. This damage is believed to result from lift of the cellular scales on the surfaces of the cuticles of the hair strands and removal of lubricating substances from the scales. The lift and de-lubrication enable breakage, dislodgement, fragmentation and disruption of the inter-scale connections of fatty acids, phospholipids and disulfide bonds. Water, soap, shampoo and similar solubilization agents are able to permeate the cuticle as a result of the lifted individual scales of the cuticle. This permeation enables dilution, solubilization and dispersion of the micro connections between the lifted scales. The result is a weakening of the hair strand cuticle leading to breakage, fragmentation and splintering of the strand.

Embodiments of the hair treatment composition according to the invention replace and reincorporate the inter-scale connections of hair strands, coat hair strand scale surfaces and close inter-scale spaces resulting from lift. The result is believed to strengthen the hair strands and render them less susceptible to lift. The strengthening can be measured by the ease of combability of such treated hair strands.

In one embodiment, the component a) is at least one C3-C6 saturated or unsaturated dicarboxylic acid, whereby the saturated dicarboxylic acid is optionally substituted with 1, 2, 3 or 4 hydroxyl groups. Preferably, the at least one C3-C6 saturated or unsaturated dicarboxylic acid, whereby the saturated dicarboxylic acid is optionally substituted with 1, 2, 3 or 4 hydroxyl groups, is selected from the group consisting of malonic acid, succinic acid, glutaric acid, adipic acid, malic acid, 2-methyl malic acid, hydroxyglutaric acid, fumaric acid, maleic acid, itaconic acid, glutaconic acid, tartaric acid, 2-methyltartaric acid, 2-hydroxy-3-methylglutaric acid, hydroxyadipic acid and hydroxyglutaric acid.

In another embodiment, the component a) is at least one C3-C6 saturated dicarboxylic acid which is substituted with 1, 2, 3 or 4 hydroxyl groups. Preferably, the at least one C3-C6 saturated dicarboxylic acid which is substituted with 1, 2, 3 or 4 hydroxyl groups is selected from the group consisting of malic acid, 2-methyl malic acid, hydroxyglutaric acid, tartaric acid, 2-methyltartaric acid, 2-hydroxy-3-methylglutaric acid, hydroxyadipic acid, and hydroxyglutaric acid.

In one embodiment, the component a) is preferably present at a weight percentage concentration in the range of from 0.1 wt.% to 10.0 wt.%, more preferably at a weight percentage concentration in the range of from 0.2 wt.% to 5.0 wt.%.

In one embodiment, the component b) is preferably at least one C16-C22 saturated or unsaturated monocarboxylic acid. In another embodiment, the at least one C16-C22 saturated or unsaturated monocarboxylic acid is selected from the group consisting of palmitic acid, stearic acid, arachidic acid, behenic acid, palmitoleic acid, sapienic acid, oleic acid, elaidic acid, vaccenic acid and linoleic acid. In another embodiment, the at least one C16-C22 monocarboxylic acid is monounsaturated. In another embodiment, the component b) is palmitoleic acid or oleic acid, in particular the component b) is oleic acid.

Each of the dicarboxylic acids and the monocarboxylic acids of the hair treatment composition may independently be in the free carboxylic acid form or may be a cosmetically acceptable salt thereof in which the counterion may be the alkali or alkaline earth metal cations that do not cause precipitation of the salt form, preferably including the sodium, potassium, lithium, magnesium, zinc, copper and aluminum cations, more preferably sodium, potassium and lithium, especially more preferably sodium and potassium.

The component b) is present at a weight percentage concentration in the range of 0.05 wt.% to 1.0 wt.%, more preferably in the of 0.05 wt.% to 0.5 wt.%.

The concentration ranges of the dicarboxylic acid, i.e., the component a), and the monocarboxylic acid, i.e., the component b), follow a general relationship in which the weight percentage relative to the total weight of the composition is larger for the dicarboxylic acid than for the monocarboxylic acid. The ratio of dicarboxylic acid to monocarboxylic acid ranges from about 2:1 to about 10:1, preferably about 2:1 to about 5:1.

In one embodiment, the at least one C10-C50 saturated or unsaturated hydrocarbon is selected from the group consisting of paraffins and saturated acyclic terpanes.

In an embodiment, the at least one saturated acyclic terpane is a monoterpane, a sesquiterpane, a diterpane, a sesterterpane, a triterpane or a tetraterpane. In another embodiment, the at least one saturated acyclic terpane is selected from the group consisting of 2,6-dimethyl octane, 2,6,10,15,19-pentamethylleicosane, farnesane, phytane, squalane, isosqualane, neosqualane and lycopane. In yet another embodiment, the at least one saturated acyclic terpane is selected from the group consisting of squalane, isosqualane and neosqualane, preferably the at least one saturated acyclic terpane is squalane. As used herein, "squalane" refers to an oil and is also referred to as 2,6,10,15,19,23-hexamethyl tetracosane. In one embodiment, the squalane is synthetic squalane which is obtained by chemical synthesis or semisynthetic squalane which is obtained from naturally occurring squalene by reduction. Squalene is present in deep sea fish, such as sharks, or olive oil, rice bran oil, wheat germ oil, sesame oil and cotton seed oil. Squalane can also be obtained from sugar cane.

In a preferred embodiment, the hair treatment composition comprises squalane derived from a bio-based farnesene or squalane, isosqualane and neosqualane derived from a bio-based farnesene, i.e., the squalane, isosqualane and neosqualane which is produced from bio-based farnesene by catalytic reaction, chemical reaction, thermal reaction, hydrogenation or any combination thereof.

As used herein, the term "bio-based farnesene" refers to farnesene which is biologically produced from microorganisms, in particular genetically modified microorganisms, by fermentation of renewable carbon sources such as sugar. As used herein, "farnesene" refers to α-farnesene, β-farnesene or a mixture thereof. α -farnesene refers to a compound having the following structure: 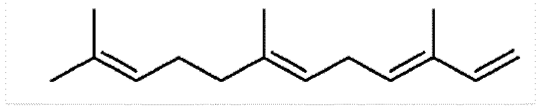 stereoisomer or a mixture of stereoisomers thereof.

β -farnesene refers to a compound having the following structure: 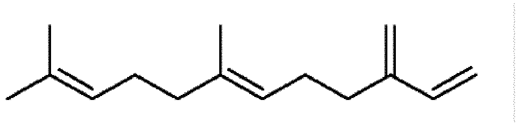 stereoisomer or a mixture thereof.

In one embodiment, β-farnesene is a substantially pure stereoisomer of β-farnesene. In another embodiment, β-farnesene comprises a mixture of stereoisomers, such as cis-trans isomers. In another embodiment, the amount of each of the stereoisomers in the β-farnesene mixture is preferably in the range of ≥ 0.1 wt.% to ≤ 99.9 wt.%, more preferably in the range of ≥ 1.0 wt.% to ≤ 99.0 wt. %, even more preferably in the range of ≥ 5.0 wt.% to ≤ 95.0 wt.%, yet more preferably in the range of ≥ 10 wt.% to ≤ 90 wt.%, most preferably in the range of ≥ 20 wt.% to ≤ 80 wt.%, based on the total weight of the β-farnesene mixture.

In one embodiment, the at least one saturated acyclic terpane has a degree of branching of 2, 3, 4, 5, 6 or 8, preferably the at least one saturated acyclic terpane has a degree of branching of 6.

In yet another embodiment, the at least one saturated acyclic terpane, preferably squalane, is preferably present in an amount in the range of ≥ 0.01 wt.% to ≤ 1.0 wt.%, more preferably in the range of ≥ 0.02 wt.% to ≤ 0.8 wt. %, even more preferably in the range of ≥ 0.03 wt.% to ≤ 0.7 wt.%, based on the total weight of the hair treatment composition.

In yet another embodiment, the paraffins are preferably present in an amount in the range of ≥ 1.0 wt.% to ≤ 6.0 wt.%, more preferably in the range of ≥ 1.5 wt.% to ≤ 5.0 wt.%, even more preferably in the range of ≥ 2.0 wt.% to ≤ 4.5 wt.%, based on the total weight of the hair treatment composition.

In one embodiment, the fatty acid ester is a fatty acid ester of a C16-C18 saturated or unsaturated monocarboxylic acid which is esterified with a C3-C6 mono-ol (a monoalcohol), a diol or a triol, preferably a triol, more preferably a C3 or C4 triol, and most preferably glycerin. A preferred fatty acid ester is glyceryl mono-palmitoleate, glyceryl monooleate or a combination thereof with glyceryl monooleate alone being more preferred.

In one embodiment, the component d) is preferably present at a weight percentage concentration in the range of from 0.15 wt.% to 3.0 wt.%, more preferably at a weight percentage concentration in the range of from 0.15 wt.% to 2.5 wt.%, even more preferably at a weight percentage concentration in the range of from 0.15 wt.% to 2.0 wt.%, in particular at a weight percentage concentration in the range of from 0.15 wt.% to 2.0 wt.%.

Preferably, when the hair treatment composition comprises a component of a sulfate and/or a sulfate-free shampoo, the lipid component may optionally comprise two versions of the fatty acid ester including the mono fatty acid ester formed with a triol and a di fatty acid ester formed with a triol. When the hair treatment composition comprises a component of a sulfate free shampoo, the lipid component may optionally include the hydrocarbon as a substitute for the di fatty acid ester of the mono and di fatty acid ester combination. When the hair treatment composition comprises a stand-alone conditioner, a mask, a leave on treatment, a rinse off conditioner, a hair rinse, a hair liquid, a hair oil, a hair pomade, a hair foam, a hair spray, a hair tonic and/or hair lotion, the lipid component may be both of a mono fatty acid ester formed with a triol and a hydrocarbon.

Embodiments of the presently claimed invention incorporate not only the organic acid component but also organic acidifying and basifying agents such as citric acid, acetic acid, tartaric acid, hydrochloric acid and alkali metal hydroxides such as sodium or potassium hydroxide. These agents enable balance of the pH of a composition of the presently claimed invention so that it is not an irritant to humans such as human scalp and/or face. Consequently, the acidifying and basifying agents may be adjusted so that the pH is in the range of about 5 to about 9, preferably in a range of about 6 to about 8.

Additional components for embodiments of the hair treatment composition depend at least in part upon the purpose and function of the hair treatment composition. For example, if the hair treatment composition is to be a part of a shampoo, anionic and/or nonionic surfactants may be included. If the hair treatment composition is to be a part of a rinse off or leave on conditioner, cationic surfactants and/or cationic polymers may be included. Moisturizers, humectants, thickeners, emulsifiers as well as other additives for delivering advantageous qualities may be included the hair treatment composition.

The hair treatment composition of the presently claimed invention may comprise a cationic surfactant, an optional silicone component and aqueous carrier. The surfactants, silicones and the aqueous carrier may be in the form of emulsion.

The composition of the presently claimed invention may optionally comprise a cationic surfactant system especially when the composition of the presently claimed invention is to deliver conditioning properties. The cationic surfactant system may comprise one cationic surfactant or a mixture of two or more cationic surfactants. When present, the cationic surfactant system may be included in the composition at a level by weight of from about 0.1% to about 10%, preferably from about 0.5% to about 8%, more preferably from about 0.8% to about 5%, still more preferably from about 1.0% to about 4% relative to the total weight of the composition. Embodiments of cationic surfactants useful in the hair treatment composition of the invention include common and ordinary quaternary ammonium salts of C14-C30 linear alkyl amines quaternized with three methyl groups. Examples include behentrimonium chloride, stearyl trimonium chloride and similar trimonium compounds.

In a preferred embodiment, the hair treatment composition, preferably the conditioner, of the presently claimed invention comprises at least one cationic surfactant selected from the group consisting of cetrimonium halide, stearimonium halide, behentrimonium halide, dodecyltrimethylammonium halide, didodecyldimethylammonium halide, tetradecyltrimethylammonium halide, distearyldimethylammonium halide, dicetyldimethylammonium halide, behenamidopropyldimethylamine, behenamidopropyldiethylamine, behenamidoethyldiethyl-amine, behenamidoethyldimethylamine, arachidamidopropyldimethylamine, arachidamido-propyidiethylamine, arachidamidoethyidiethylamine and arachidamidoethyidimethylamine.

The hair treatment composition of the presently claimed invention may also comprise one or more silicone conditioning agents. Examples of the silicones include dimethicones, dimethiconols, cyclic silicones, and modified silicones with various functional groups such as amino groups, quaternary ammonium salt groups, and amine silicones such as Amodimethicone. Such silicones may be soluble or insoluble in the aqueous (or aqueous-organic) medium. In the case of insoluble liquid silicones, the polymer can be in an emulsified form with droplet size of about 10 nm to about 30 micrometers. Other solid or semi-solid conditioning agents may be present in the composition including high melting temperature fatty alcohols, acids, esters, amides or oligomers from unsaturated esters, alcohols, amides. The oligomeric esters may be the result of oligomerization of naturally-occurring unsaturated glyceride esters. Such solid or semi-solid conditioning agents may be added or present as mixtures with organic oils.

In one embodiment, the hair treatment composition is a shampoo comprising
a) at least one C3-C13 saturated or unsaturated dicarboxylic acid which is unsubstituted or substituted with 1, 2, 3 or 4 hydroxyl groups at a weight percentage concentration of from 0.2 wt.% to 4.0 wt.%;
b) at least one C10-C30 saturated or unsaturated monocarboxylic acid at a weight percentage concentration of 0.05 wt.% to 1.0 wt.%;
c) at least one C10-C50 saturated or unsaturated hydrocarbon at a weight percentage concentration of 0.01 wt.% to 0.5 wt.%;
d) at least one fatty acid ester of a C10-C30 saturated or unsaturated monocarboxylic acid and a C3-C6 mono-ol, diol or triol at a weight percentage concentration of 0.1 wt.% to 1.0 wt.%;
e) at least one cosmetically acceptable aqueous or aqueous-organic medium;
wherein the components a) and b) are present in the form of a free acid or a cosmetically acceptable salt thereof and all weight percentages are relative to the total weight of the composition.

In a preferred embodiment, the hair treatment composition is a shampoo comprising
a) malic acid at a weight percentage concentration of from 0.2 wt.% to 4.0 wt.%;
b) oleic acid at a weight percentage concentration of 0.05 wt.% to 1.0 wt.%;
c) squalane at a weight percentage concentration of 0.01 wt.% to 0.2 wt.%;
d) glyceryl monooleate a weight percentage concentration of 0.1 wt.% to 0.5 wt.%;
e) at least one cosmetically acceptable aqueous or aqueous-organic medium;
wherein the components a) and b) are present in the form of a free acid or a cosmetically acceptable salt thereof and all weight percentages are relative to the total weight of the composition.

In one embodiment, the hair treatment composition is a conditioner comprising
a) at least one C3-C13 saturated or unsaturated dicarboxylic acid which is unsubstituted or substituted with 1, 2, 3 or 4 hydroxyl groups at a weight percentage concentration of from 0.2 wt.% to 5.0 wt.%;
b) at least one C10-C30 saturated or unsaturated monocarboxylic acid at a weight percentage concentration of 0.05 wt.% to 1.0 wt.%;
c) at least one C10-C50 saturated or unsaturated hydrocarbon at a weight percentage concentration of 0.01 wt.% to 10.0 wt.%;
d) at least one fatty acid ester of a C10-C30 saturated or unsaturated monocarboxylic acid and a C3-C6 mono-ol, diol or triol at a weight percentage concentration of 0.1 wt.% to 3.0 wt.%;
e) at least one cosmetically acceptable aqueous or aqueous-organic medium;
wherein the components a) and b) are present in the form of a free acid or a cosmetically acceptable salt thereof and all weight percentages are relative to the total weight of the composition.

In a preferred embodiment, the hair treatment composition is a conditioner comprising
a) malic acid at a weight percentage concentration of from 0.2 wt.% to 5.0 wt.%;
b) oleic acid at a weight percentage concentration of 0.05 wt.% to 1.0 wt.%;
c) at least one C10-C50 saturated or unsaturated hydrocarbon selected from the group consisting of squalane and paraffins at a weight percentage concentration of 0.01 wt.% to 10.0 wt.%;
d) glyceryl monooleate a weight percentage concentration of 0.2 wt.% to 2.0 wt.%;
e) at least one cosmetically acceptable aqueous or aqueous-organic medium;
wherein the components a) and b) are present in the form of a free acid or a cosmetically acceptable salt thereof and all weight percentages are relative to the total weight of the composition.

In one embodiment, the hair treatment composition is a leave-on treatment comprising
a) at least one C3-C13 saturated or unsaturated dicarboxylic acid which is unsubstituted or substituted with 1, 2, 3 or 4 hydroxyl groups at a weight percentage concentration of from 0.1 wt.% to 1.0 wt.%;
b) at least one C10-C30 saturated or unsaturated monocarboxylic acid at a weight percentage concentration of 0.05 wt.% to 1.0 wt.%;
c) at least one C10-C50 saturated or unsaturated hydrocarbon at a weight percentage concentration of 0.05 wt.% to 1.0 wt.%;
d) at least one fatty acid ester of a C10-C30 saturated or unsaturated monocarboxylic acid and a C3-C6 mono-ol, diol or triol at a weight percentage concentration of 0.1 wt.% to 1.0 wt.%;
e) at least one cosmetically acceptable aqueous or aqueous-organic medium;
wherein the components a) and b) are present in the form of a free acid or a cosmetically acceptable salt thereof and all weight percentages are relative to the total weight of the composition.

In a preferred embodiment, the hair treatment composition is a leave-on treatment comprising
a) malic acid at a weight percentage concentration of from 0.1 wt.% to 1.0 wt.%;
b) oleic acid at a weight percentage concentration of 0.05 wt.% to 1.0 wt.%;
c) squalane at a weight percentage concentration of 0.05 wt.% to 1.0 wt.%;
d) glyceryl monooleate at a weight percentage concentration of 0.1 wt.% to 1.0 wt.%;
e) at least one cosmetically acceptable aqueous or aqueous-organic medium;
wherein the components a) and b) are present in the form of a free acid or a cosmetically acceptable salt thereof and all weight percentages are relative to the total weight of the composition.

When embodiments of the hair treatment composition are formulated as a shampoo, the shampoo will include a surfactant such as a sulfate or non-sulfate anionic surfactant or a non-ionic surfactant or any combination thereof. According to the invention, embodiments of surfactant component of the shampoo composition may comprise at least one member of the anionic surfactant class including a sulfate anionic surfactant or a non-sulfate anionic surfactant. The anionic surfactant class may also include multiple species of each member as well as a combination of these class members and any combination of multiple species of both members.

Additionally, the surfactant component may further comprise a non-ionic surfactant in combination with any variation of the anionic surfactant class described above. Preferably, the surfactant component may comprise one or more sulfate anionic surfactants alone, one or more non-sulfate anionic surfactants alone, a mixture of a minor amount of one or more sulfate anionic surfactants and a major amount of one or more non-sulfate anionic surfactants, a mixture of one or more sulfate anionic surfactants and one or more non-ionic surfactants, or a mixture of one or more non-sulfate anionic surfactants and one or more non-ionic surfactants. More preferably, the surfactant component may comprise one or more non-sulfate anionic surfactants alone. Also preferably, the surfactant component may comprise one or more non-sulfate anionic surfactants combined with one or more non-ionic surfactants. Especially more preferably the surfactant component may comprise a combination of two members of the surfactant class such as, but not limited to, a combination of one or more sulfate-free non-sulfate anionic surfactants and one or more non-ionic surfactants.

Another especially preferred surfactant class may comprise any of the embodiments including a non-sulfate anionic surfactant which may be devoid of any sulfate anionic surfactant. This surfactant class may be characterized as a sulfate-free non-sulfate anionic surfactant. Without the characterization of "sulfate free", a surfactant component comprising a non-sulfate anionic surfactant and/or a non-ionic surfactant may include but not necessarily include a minor concentration to an almost indetectable concentration of sulfate anionic surfactant which may be incidentally or purposefully present.

The sulfate anionic surfactant class of the surfactant component of the present shampoo composition may comprise a branched and/or linear C8 to C20 alkyl sulfate, a linear and/or branched C8 to C20 alkyl benzene sulfate or a linear and/or branched C8 to C20 alkyl benzyl sulfate or the corresponding ether and/or glyceryl and/or sugar alcohol derivatives obtained from ethylene epoxide/ethylene glycol, propylene epoxide/1,2-propylene glycol, glycerin and/or sugar alcohol monomers. The linear and/or branched alkyl group may be a single chain length or may be a mixture of chain lengths.

Exemplary sulfate anionic surfactants comprise one or more of sodium lauryl sulfate, sodium laureth sulfate, ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric mono glyceride sodium sulfate, potassium lauryl sulfate, potassium laureth sulfate, ammonium cocoyl sulfate, sodium cocoyl sulfate, potassium cocoyl sulfate, monoethanolamine cocoyl sulfate, monoethanolamine lauryl sulfate, sodium tridecyl benzene sulfate and sodium dodecyl benzene sulfate.

The non-sulfate anionic surfactant class of the surfactant component of the present shampoo composition may be selected from the group of acyl isethionates/methyl isethionates, acyl glycinates, acyl taurates, acyl amino acids, acyl sarcosinates, sulfosuccinates and sulfonates, wherein the acyl groups of all these surfactant classes comprise from 6 to 30 carbon atoms. The non-sulfate anionic surfactant may also comprise more than one of these surfactants and may comprise mixtures thereof. The non-sulfate anionic surfactant segment may also comprise the alkaline and alkaline earth salts of one or more or mixtures of individual compounds of any of these surfactant classes and mixtures thereof.

In a preferred embodiment, the hair treatment composition, preferably the shampoo, of the presently claimed invention comprises at least one alkanolamide surfactants selected from the group consisting of acetamide MEA, cocamide MEA, cocamide DEA, cocamide methyl MEA, cocamide MIPA, hydroxystearamide MEA, PEG-5 cocamide MEA, lactamide MEA, lauramide MEA and lauramide DEA.

Fatty alcohols may optionally be included in embodiments of the shampoo and conditioning hair treatment compositions of the invention. The fatty alcohols provide emulsifying, occlusive, moisturizing, thickening and humectant properties to the hair treatment composition. Included are fatty alcohols and mixtures thereof having a linear or branched carbon chain of from about 12 to about 30 carbons. The fatty alcohols may be saturated or unsaturated. Preferred fatty alcohols include lauryl alcohol, myristyl alcohol, cetyl alcohol, stearyl alcohol, behenyl alcohol, oleyl alcohol, arachidyl alcohol and mixtures thereof such as cetearyl alcohol (mixture of cetyl and stearyl alcohols).

In a preferred embodiment, the hair treatment composition, preferably the shampoo, of the presently claimed invention comprises at least one betaine which is selected from the group consisting of alkylbetaines, alkylamidopropylbetaines and alkylsulfobetaines.

In a preferred embodiment, the hair treatment composition, preferably the shampoo, of the presently claimed invention comprises at least one alkylbetaine of general formula (V),

R^{f}R^{g}R^{h}N⁺-CH₂-C(=O)-O⁻ (V),

wherein R^{f} is a substituted or unsubstituted alkyl or alkenyl group having 7 to 22 carbon atoms and R^{g} and R^{h} are each, same or different, selected from the group consisting of C1-C6 alkyl, C1-C6 hydroxyalkyl and C1-C6 carboxyalkyl. R^{f} is preferably a mixture of C12 and C14 alkyl groups derived from coconut so that at least half, preferably at least three quarters, of the groups R^{f} have 10 to 14 carbon atoms. R^{g} and R^{h} are preferably methyl.

In a preferred embodiment, the at least one betaine is selected from the group consisting of coco betaine, cocoamidopropyl betaine, lauryl betaine, laurylhydroxy sulfobetaine, lauryldimethyl betaine, cocoamidopropylhydroxysulfo betaine, behenyl betaine, capryl betaine, capramidopropyl betaine and stearyl betaine, more preferably the at least one betaine (D) is cocoamidopropyl betaine.

In a preferred embodiment, the at least one betaine is present in the cleansing composition of the presently claimed invention in an amount in the range of ≥ 2.0 to ≤ 10.0 wt.%, more preferably in the range of ≥ 3.0 to ≤ 9.0 wt.%, even more preferably in the range of ≥ 4.0 to ≤ 8.0 wt.%, based on the total weight of the hair treatment composition.

In a preferred embodiment, the hair treatment composition, preferably the shampoo, of the presently claimed invention comprises at least one conditioning agent. The conditioning agent is preferably a cationic or ampholytic conditioning agent.

In a preferred embodiment, the at least one conditioning agent is a cationic cellulose. Cationic cellulose comprises salts of hydroxyethyl cellulose reacted with trimethyl ammonium substituted epoxide, referred to in the industry as polyquaternium-10. Another type of cationic cellulose includes the polymeric quaternary ammonium salts of hydroxyethyl cellulose reacted with lauryl dimethyl ammonium-substituted epoxide, referred to in the industry as polyquaternium-24.

In another preferred embodiment, the at least one conditioning agent is a cationic polysaccharide polymer, especially a cationic guar gum derivative, such as guar hydroxypropyltrimonium chloride which is commercially available from Solvay in their JAGUAR trademark series.

In a preferred embodiment, the at least one conditioning agent is selected from the group consisting of polyquaternium-1, polyquaternium-2, polyquaternium-4, polyquaternium-5, polyquaternium-6, polyquaternium-7, polyquaternium-8, polyquaternium-9, polyquaternium-10, polyquaternium-11, polyquaternium-12, polyquaternium-13, polyquaternium-14, polyquaternium-15, polyquaternium-16, polyquaternium-17, polyquaternium-18, polyquaternium-19, polyquaternium-20, polyquaternium-22, polyquaternium-24, polyquaternium-27, polyquaternium-28, polyquaternium-29, polyquaternium-30, polyquaternium-31, polyquaternium-32, polyquaternium-33, polyquaternium-34, polyquaternium-35, polyquaternium-36, polyquaternium-37, polyquaternium-38, polyquaternium-39, polyquaternium-43, polyquaternium-44, polyquaternium-45, polyquaternium-46, polyquaternium-47, polyquaternium-48, polyquaternium-49, polyquaternium-50, polyquaternium-52, polyquaternium-53, polyquaternium-54, polyquaternium-55, polyquaternium-56, polyquaternium-57, polyquaternium-58, polyquaternium-59, polyquaternium-60, polyquaternium-63, polyquaternium-64, polyquaternium-65, polyquaternium-66, polyquaternium-67, polyquaternium-70, polyquaternium-73, polyquaternium-74, polyquaternium-75, polyquaternium-76, polyquaternium-85, polyquaternium-86, polybeta-alanine, polyepsilon-lysine, polylysine, PEG-8/SMDI copolymer, PPG-12/SMDI copolymer, PPG-51/SMDI copolymer, PPG-7/succinic acid copolymer, IPDI/PEG-15 cocamine copolymer, IPDI/PEG-15 cocamine copolymer dimer dilinoleate, IPDI/PEG-15 soyamine copolymer, IPDI/PEG-15 soyamine oxide copolymer, IPDI/PEG-15 soyethonium ethosulfate copolymer, polyquaternium-4/hydroxypropyl starch copolymer, cassia hydroxypropyltrimonium chloride, chitosan hydroxypropyltrimonium chloride, dextran hydroxypropyltrimonium chloride, galactoarabinan hydroxypropyltrimonium chloride, ginseng hydroxypropyltrimonium chloride, guar hydroxypropyltrimonium chloride, hydroxypropyl guar hydroxypropyltrimonium chloride, locust bean hydroxypropyltrimonium chloride, starch hydroxypropyltrimonium chloride, hydroxypropyltrimonium hydrolyzed corn starch, hydroxypropyl oxidized starch PG-trimonium chloride, tamarindus indica hydroxypropyltrimonium chloride, polyacrylamidopropylltrimonium chloride, polymethacrylamidopropylltrimonium chloride, polyacrylamidopropylltrimonium methosulfate, propyltrimoniumchloride methacrylamide/dimethylacrylamide copolymer, acrylamide/ethalkonium chloride acrylate copolymer, acrylamide/ethyltrimonium chloride acrylate/ethalkonium chloride acrylate copolymer, acrylates/carbamate copolymer, adipic acid/methyl DEA crosspolymer, diethylene glycol/DMAP acrylamide/PEG-180/HDI copolymer, dihydroxyethyl tallowamine/IPDI copolymer, dimethylamine/ethylenediamine/epichlorohydrin copolymer, HEMA glucoside/ethylmethacrylate trimonium chloride copolymer, hydrolyzed wheat protein/PEG-20 acetate copolymer, hydrolyzed wheat protein/PVP crosspolymer, ethyltrimonium chloride methacrylate/hydroxyethylacrylamide copolymer; more preferably the at least one component (E) is selected from the group consisting of polyquaternium-10 and guar hydroxypropyltrimonium chloride.

In a preferred embodiment, the at least one conditioning agent is present in the hair treatment composition, preferably the shampoo, of the presently claimed invention in an amount in the range of ≥ 0.1 to ≤ 1.0 wt.%, more preferably in the range of ≥ 0.1 to ≤ 0.9 wt.%, even more preferably in the range of ≥ 0.1 to ≤ 0.8, 0.7, 0.6. or 0.5 wt.%, based on the total weight of the hair treatment composition, preferably the shampoo.

The hair treatment composition of the presently claimed invention may comprise an aqueous carrier as cosmetically acceptable medium. Accordingly, embodiments of the compositions of the presently claimed invention can be in the form of pourable liquids (under ambient conditions). Such compositions will therefore typically comprise an aqueous carrier, which may be present at a level of from about 20 wt. % to about 95 wt. %, or from about 60 wt. % to about 85-90 wt. %. The aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

The aqueous carrier useful in the presently claimed invention may alternatively comprise water solutions of lower alkyl alcohols and polyhydric alcohols. The lower alkyl alcohols useful herein are monohydric alcohols having 1 to 6 carbons, in one aspect, ethanol and isopropanol. The polyhydric alcohols useful herein include propylene glycol, hexylene glycol, glycerin, and propane diol. Aqueous-organic carriers for the hair treatment compositions of the invention may comprise from about 50 wt.% to about 99 wt.% water, preferably about 75 wt.% to about 95 wt.% water, more preferably about 85 wt.% to about 95 wt.% water with the remainder being a mono or polyhydric alcohol, preferably any one or a mixture of any two or more of ethanol, isopropanol, propylene glycol and glycerin.

The compositions of the presently claimed invention can also additionally comprise any other suitable optional ingredients as desired. For example, the composition can optionally include other active or inactive ingredients known for scalp health benefits.

In an embodiment of the presently claimed invention, a scalp health active may be added to provide scalp benefits. This group of scalp health materials is varied and provides a wide range of benefits including anti-dandruff, anti-fungal, anti-microbial, moisturization, barrier improvement, and anti-oxidant, anti-itch, and sensates. Such health actives include but are not limited to: zinc pyrithione, climbazole, octopirox, vitamin E and F, salicylic acid, phenoxyethanol, ethylhexyl glycerin, glycols, glycolic acid, PCA, PEGs, erythritol, glycerin, lactates, hyaluronates, allantoin and other ureas, betaines, sorbitol, glutamates, xylitols, menthol, menthyl lactate, isocyclomone, benzyl alcohol, and natural extracts/oils including peppermint, spearmint, argan, jojoba and aloe. The compositions may include other common hair ingredients. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the composition herein. Examples of these ingredient classes include but are not limited to: aesthetic components such as fragrances, pigments, colorings/colorants, essential oils, sensates, antifoaming agents, antimicrobial agents, biological additives, buffering agents, bulking agents, chelating agents, chemical additives, astringents, biocides, film formers or materials, pH adjusters, reducing agents, sequestrants, and surfactants.

In a preferred embodiment, the hair treatment composition, preferably the conditioner, of the presently claimed invention comprises at least one liquid oil.

For example, the liquid oil may be selected from the group consisting of avocado oil, tsubaki oil, turtle oil, macadamia nuts oil, corn oil, mink oil, olive oil, rape seed oil, yolk oil, sesame oil, parsic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, jojoba oil, germ oil, triglycerine, trioctanoic acid glycerine, triisopalmitic acid glycerine, liquid lanolin, hexyl laurate, liquid hydrocarbon esters such as fluid paraffin, mineral oil, isododecane, fatty acid oils, ester oils such as cetyl octanoate, isopropyl myristate and mixtures thereof. The hair treatment composition may be applied neat to anagenic hair (hair on the head of a human) or may be diluted with water or a combination of water and a water miscible alcohol and applied to anagenic hair. After application, the hair treatment composition on the hair may be massaged and worked through the hair to distribute the composition throughout the hair. The Following a sufficient time to accomplish cleaning, conditioning, and/or other function of the hair treatment composition on the hair, the hair may be rinsed with water or if the hair treatment composition is a leave on, the hair may be troweled briefly to remove excess composition if any.

### Embodiments

In the following, there is provided a list of embodiments to illustrate the present disclosure without intending to limit the disclosure to the specific embodiments listed below.
1. A hair treatment composition comprising:
   a) at least one C3-C13 saturated or unsaturated dicarboxylic acid which is unsubstituted or substituted with 1, 2, 3 or 4 hydroxyl groups at a weight percentage concentration of from 0.05 wt.% to 15.0 wt.%;
   b) at least one C10-C30 saturated or unsaturated monocarboxylic acid at a weight percentage concentration of 0.05 wt.% to 1.0 wt.%;
   c) at least one C10-C50 saturated or unsaturated hydrocarbon at a weight percentage concentration of 0.01 wt.% to 10.0 wt.%;
   d) at least one fatty acid ester of a C10-C30 saturated or unsaturated monocarboxylic acid and a C3-C6 mono-ol, diol or triol at a weight percentage concentration of 0.1 wt.% to 5.0 wt.%;
   e) at least one cosmetically acceptable aqueous or aqueous-organic medium;
   wherein the components a) and b) are present in the form of a free acid or a cosmetically acceptable salt thereof and
   all weight percentages are relative to the total weight of the composition.
2. The hair treatment composition according to embodiment 1, wherein the component a) is at least one C3-C6 saturated or unsaturated dicarboxylic acid which is unsubstituted or substituted with 1, 2, 3 or 4 hydroxyl groups.
3. The hair treatment composition according to embodiment 2, wherein the at least one C3-C6 saturated or unsaturated dicarboxylic acid which is unsubstituted or substituted with 1, 2, 3 or 4 hydroxyl groups is selected from the group consisting of malonic acid, succinic acid, glutaric acid, adipic acid, malic acid, 2-methyl malic acid, hydroxyglutaric acid, fumaric acid, maleic acid, itaconic acid, glutaconic acid, tartaric acid, 2-methyltartaric acid, 2-hydroxy-3-methylglutaric acid, hydroxyadipic acid and hydroxyglutaric acid.
4. The hair treatment composition according to embodiment 2, wherein the component a ) is at least one C3-C6 saturated dicarboxylic acid which is substituted with 1, 2, 3 or 4 hydroxyl groups.
5. The hair treatment composition according to embodiment 4, wherein the at least one C3-C6 saturated dicarboxylic acid which is substituted with 1, 2, 3 or 4 hydroxyl groups is selected from the group consisting of malic acid, 2-methyl malic acid, hydroxyglutaric acid, tartaric acid, 2-methyltartaric acid, 2-hydroxy-3-methylglutaric acid, hydroxyadipic acid, and hydroxyglutaric acid.
6. The hair treatment composition according to any of the preceding embodiments, wherein the component b) is at least one C16-C22 saturated or unsaturated monocarboxylic acid.
7. The hair treatment composition according to embodiment 6, wherein the component b) is selected from the group consisting of oleic acid and palmitoleic acid.
8. The hair treatment composition according to any of the preceding embodiments, wherein the component c) is selected from the group consisting of paraffins and saturated acyclic terpanes.
9. The hair treatment composition according to embodiment 8, wherein the saturated acyclic terpanes are selected from the group consisting of squalane, isosqualane and neosqualane.
10. The hair treatment composition according to any of the preceding embodiments, wherein the component d) is at least one fatty acid ester of a C16-C22 saturated or unsaturated monocarboxylic acid and a C3-C6 triol.
11. The hair treatment composition according to any of the preceding embodiments, wherein the component a) is malic acid and the component b) is oleic acid.
12. The hair treatment composition according to any of the preceding embodiments, wherein
   the component a) is selected from the group consisting of malic acid and glutaric acid,
   the component b) is selected from the group consisting of oleic acid and palmitoleic acid,
   the component c) is selected from the group consisting of squalane and paraffins, and
   the component d) is selected from the group consisting of glyceryl monooleate and glyceryl mono-palmitoleate.
13. The hair treatment composition according to embodiment 12, wherein
   the component a) is malic acid,
   the component b) is oleic acid,
   the component c) is squalane,
   the component d) is glyceryl monooleate.
14. The hair treatment composition according to any of the preceding embodiments, wherein the hair treatment composition comprises at least one additive selected from the group consisting of cationic surfactants, silicone conditioners, anionic surfactants, bleaching agents, coloring agents, and permanent hair waving agents.
15. The hair treatment composition according to any of the preceding embodiments, wherein the component a) is present at a weight percentage concentration in the range of from 0.1 wt.% to 10.0 wt.%.
16. The hair treatment composition according to any of the preceding embodiments, wherein the component c) is present at a weight percentage concentration in the range of 0.02 wt.% to 4.0 wt.%.
17. The hair treatment composition according to any of the preceding embodiments, wherein the component d) is present at a weight percentage concentration in the range of 0.15 wt.% to 3.0 wt.%.
18. The hair treatment composition according to any of the preceding embodiments, wherein the ratio of the weight percentage concentration of the component a) to the weight percentage concentration of the component b) is in the range of 10.0:1.0 to 1.0:1.0, preferably in the range of 5.0:1.0 to 2.0:1.0.
19. The hair treatment composition according to any of the preceding embodiments, wherein the component e) is selected from the group consisting of water and C2-C6 mono-ols.
20. The hair treatment composition according to any of the preceding embodiments, wherein the pH of the hair treatment composition is in the range from 4.0 to 6.0.
21. The hair treatment composition according to any of the preceding embodiments, wherein the hair treatment composition is selected from the group consisting of hair conditioners, hair creams, hair shampoos, dry shampoos, hair rinses, hair liquids, hair oils, hair pomades, hair foams, hair sprays, hair tonics and hair lotions.
22. A method for treating hair comprising at least the step of A) applying to the hair a hair treatment composition according to any of embodiments 1 to 21.
23. The method according to embodiment 22 further comprising the step of B) diluting the hair treatment composition according to any of embodiments 1 to 21 with water before or while applying to the hair.
24. The use of the hair treatment composition according to any of embodiments 1 to 21 for repairing damaged hair.

### Statements:

X1. A hair treatment composition comprising:
   a) at least one C3-C13 saturated or unsaturated dicarboxylic acid, which is unsubstituted or substituted with one hydroxyl group, at a weight percentage concentration of from 0.05 wt% to 15.0 wt%;
   b) at least one C10-C30 saturated or unsaturated monocarboxylic acid at a weight percentage concentration of 0.05 wt% to 1.0 wt%;
   c) at least one C10-C40 saturated or unsaturated hydrocarbon at a weight percentage concentration of 0.01 wt% to 1.0 wt%;
   d) at least one fatty acid ester of a C10-C30 saturated or unsaturated monocarboxylic acid and a C3-C6 mono-ol, diol or triol at a weight percentage concentration of 0.1 wt% to 10.0 wt%;
   e) at least one cosmetically acceptable aqueous or aqueous-organic medium; wherein the components a) and b) are present in the form of a free acid or a cosmetically acceptable salt thereof and
   all weight percentages are relative to the total weight of the composition.
X2. The hair treatment composition according to statement X1 wherein the component a) is at least one C3-C6 saturated or unsaturated dicarboxylic acid, the saturated dicarboxylic acid being optionally substituted with 1, 2, 3 or 4 hydroxyl groups.
X3. The hair treatment composition according to statements X1 or X2 wherein the component a) is selected from the group consisting of malonic acid, succinic acid, glutaric acid, adipic acid, malic acid, hydroxysuccinic acid, glutaric acid, hydroxyglutaric acid, fumaric acid, maleic acid, itaconic acid, glutaconic acid, the cosmetically acceptable salts thereof, and any combination thereof.
X4. The hair treatment composition according to any of the preceding statements wherein the component b) is at least one C16-C22 saturated or unsaturated monocarboxylic acid.
X5. The hair treatment composition according to any of the preceding statements wherein the component c) is at least one fatty acid ester of a C16-C22 saturated or unsaturated monocarboxylic acid and a C3-C6 triol.
X6. The hair treatment composition according to any of the preceding statements wherein the component c) is at least one C26-C40 saturated or unsaturated hydrocarbon.
X7. The hair treatment composition according to any of the preceding statements wherein the component a) is malic acid and the component b) is oleic acid.
X8. The hair treatment composition according to any of statements X1 to X6 wherein
   the component a) is selected from the group consisting of malic acid and glutaric acid,
   the component b) is selected from the group consisting of oleic acid and palmitoleic acid,
   the component c) is selected from the group consisting of squalane and squalene,
   the component d) is selected from the group consisting of glyceryl monooleate and glyceryl mono-palmitoleate.
X9. The hair treatment composition according to statement X8 wherein
   the component a) is malic acid,
   the component b) is oleic acid,
   the component c) is squalane,
   the component d) is glyceryl monooleate.
X10. The hair treatment composition according to any of the preceding statements further comprising at least one additive selected from the group consisting of cationic surfactants, silicone conditioners, anionic surfactants, bleaching agents, coloring agents, and permanent hair waving agents.
X11. The hair treatment composition according to any of the preceding statements wherein the component a) is present at a weight percentage concentration in the range of from 0.1 wt% to 10.0 wt% and the component b) is present at a weight percentage concentration in the range of 0.05 wt% to 1.0 wt%.
X12. The hair treatment composition according to any of the preceding statements wherein the components c) and d) are present at a weight percentage concentration in the range of 0.01 wt% to 10.0 wt%.
X13. The hair treatment composition according to any of the preceding statements wherein the ratio of the weight percentage concentration of the component a) to the weight percentage concentration of the component b) is in the range of 10.0:1.0 to 1.0:1.0, preferably 5.0:1.0 to 2.0:1.0.
X14. The hair treatment composition according to any of the preceding statements wherein the component e) is selected from the group consisting of water and C2-C6 mono-ols.
X15. The hair treatment composition according to any of the preceding statements wherein the pH of the hair treatment composition is in the range from 4.0 to 6.0.
X16. The hair treatment composition according to any of the preceding statements wherein the hair treatment composition is selected from the group consisting of hair conditioners, hair creams, hair shampoos, dry shampoos, hair rinses, hair liquids, hair oils, hair pomades, hair foams, hair sprays, hair tonics and hair lotions.
X17. A method for treating hair comprising at least the step of
   A) applying to the hair a hair treatment composition according to any of statements X1 to X16.
X18. The method according to statement 15 further comprising the step of
   B) diluting the hair treatment composition according to any of statements X1 to X16 with water before or while applying to the hair.
X19. The use of the hair treatment composition according to any of statements X1 to X16 for repairing damaged hair

### EXAMPLES

The following examples illustrate the presently claimed invention. The exemplified compositions can be prepared by conventional formulation and mixing techniques. It will be appreciated that other modifications of the presently claimed invention within the skill of those in the hair care formulation art can be undertaken without departing from the scope of this invention. All parts, percentages, and ratios herein are by weight unless otherwise specified. Some components may come from suppliers as dilute solutions.

It has been surprisingly discovered that a hair conditioning composition containing a combination of at least the dicarboxylic acid, the fatty monocarboxylic acid, the fatty hydrocarbon and the fatty acid ester improves the combability of damaged hair and improves the quality of the hair cuticle.

### Combing Force Measurement

Various external influences, such as certain cosmetic treatments (bleaching, coloring, permanent waving), exposure to the weather, frequent combing and brushing affect the combing ease due to a degradation of the hair cuticle. Thus, the combing ease of hair is a major parameter for describing the quality/degree of damage of hair on the one hand and/or the effectiveness of conditioning respectively hair repair treatments on the other hand.

The principle of most methods for the determination of combing properties consists in measuring the force to pull a comb through a hair tress under definite conditions (Ref.: C.R. Robbins, Chemical and Physical Behavior of Human Hair, 5th Edition, p. 646 ff., Springer-Verlag, Berlin Heidelberg 2012. ISBN 978-3-642-25610-3).

For this test, an automated device is used in which the tresses to be tested are taken from a storage chamber and are fixed to a force meter. The tresses are combed automatically at constant speed. For each combing stroke the combing force is recorded as a function of the distance combed through (length of the tress). For comparison purposes 20 combing strokes per tress on at least 3 tresses per product sample are averaged.

The Wet Combing Force is the average force along the tress. The lower the combing force the better the combing ease.

### Hair tresses preparation for the combing force measurements

Hair tresses (Caucasian hair), weight 2g, 17cm long, medium-blonde color, are two times bleached to simulate hair damage and then soaked in city water for 10 min (35°C ± 2°C). Afterwards, the tresses are adjusted to 50% rel. humidity by weight, then combed twice (rough & fine side of comb). 0.25ml/g hair of the hair care composition to be tested are applied to the tresses and massaged in for 1 min. The tresses are rinsed off for 1 min using city water (6L/min; 35°C ± 2°C). Afterwards, the tresses are adjusted to 50% rel. humidity by weight. The tresses are combed twice (rough & fine side of comb) and placed inside a wet magazine of the combing device storage chamber for the actual wet combing force measurements.

### Examination results

The following table shows the measured wet combing forces in Newton (N) of hair swatches subjected to damage and then treated with the hair treatment composition according to the invention as well as treatment compositions lacking one or more of the four components of the hair treatment composition of the invention. The treated hair swatches subjected to the mechanical combing measurement described above is damaged hair (two times bleached) treated with a liquid composition containing the ingredients of example 3.

| **Combing Force / N** | **Sample** |
|---|---|
| 0.073 | 2x bleached + treated with example 3 (composition with lipids, fatty acid and dicarboxylic acid) |

The results indicate clearly that hair treated with compositions containing lipids plus fatty acid and dicarboxylic acid (example 3) show excellent combing (low combing forces).

### Example 1 (shampoo)

| **Raw Material** | **Amount** |
|---|---|
| Sodium Laureth Sulfate, 70% in water | 8.6 g |
| Sodium Lauryl Sulfate, 29% in water | 19.0 g |
| Cocamidopropyl Betaine, 30% in water | 6.7 g |
| Guar Hydroxypropyltrimonium Chloride | 0.2 g |
| Dimethicone 330M | 1.1 g |
| Cocamide MEA 85% | 1.0 g |
| Ethylene Glycol Distearate | 1.5 g |
| Oleic Acid | 0.1 g |
| Glyceryl Monooleate | 0.2 g |
| Malic Acid | 0.5 g |
| Squalane | 0.05 g |
| Sodium Benzoate | 0.5 g |
| Salicylic Acid | 0.2 g |
| Tetrasodium EDTA | 0.16 g |
| D, L-Tocopheryl Acetate | 0.2 g |
| Perfume | 0.7 g |
| Sodium Chloride | 0.15 g |
| Water | to 100 g |

### Example 2 (rinse-off conditioner/mask)

| **Raw Material** | **Amount** |
|---|---|
| Cetyl Alcohol | 8.0 g |
| Paraffinum Liquidum | 3.0 g |
| Isopropyl Myristate | 3.0 g |
| Cetrimonium Chloride | 1.0 g |
| Behentrimonium Chloride | 0.4 g |
| Amodimethicone | 0.3 g |
| Ceteareth-25 | 1.5 g |
| Coco-Glucoside | 0.45 g |
| Glyceryl Monooleate | 0.45 g |
| Polyquaternium-37 | 0.125 g |
| Oleic Acid | 0.1 g |
| Malic Acid | 3.0 g |
| Sodium Hydroxide | 1.0 g |
| Disodium EDTA | 0.1 g |
| Squalane | 0.1 g |
| Phenoxyethanol | 0.9 g |
| Ethylhexylglycerin | 0.1 g |
| Perfume | 0.3 g |
| Water | to 100 g |

### Example 3 (leave-on treatment)

| **Raw Material** | **Amount** |
|---|---|
| Propylene glycol | 3.0 g |
| Amodimethicone | 1.8 g |
| Quaternium-80 | 1.0 g |
| Cetrimonium Chloride | 1.0 g |
| Behentrimonium Chloride | 1.6 g |
| D, L-Tocopheryl Acetate | 0.2 g |
| Glyceryl Monooleate | 0.25 g |
| Polyquaternium-37 | 0.125 g |
| Oleic Acid | 0.1 g |
| Malic Acid | 0.3 g |
| Sodium Hydroxide | 0.1 g |
| Disodium EDTA | 0.1 g |
| Squalane | 0.1 g |
| Phenoxyethanol | 0.1 g |
| Methylparaben | 0.2 g |
| Perfume | 0.3 g |
| Water | to 100 g |

### Example 4 (composition for mixing with a bleaching agent, a coloring agent, or a permanent hair waving agent)

| **Raw Material** | **Amount** |
|---|---|
| Malic Acid | 9.0 g |
| Quaternium-80 | 2.25 g |
| Cetrimonium Chloride | 1.9 g |
| Sodium Hydroxide | 2.9 g |
| Sodium Sulfite | 0.25 g |
| Coceth-10 | 0.9 g |
| Propylene Glycol | 0.75 g |
| Butylene Glycol | 0.5 g |
| Phenoxyethanol | 0.9 g |
| PEG-35 Castor Oil | 0.3 g |
| Glyceryl Monooleate | 0.25 g |
| Oleic Acid | 0.1 g |
| Squalane | 0.1 g |
| Water | to 100 g |

### Example 5 (leave-on treatment)

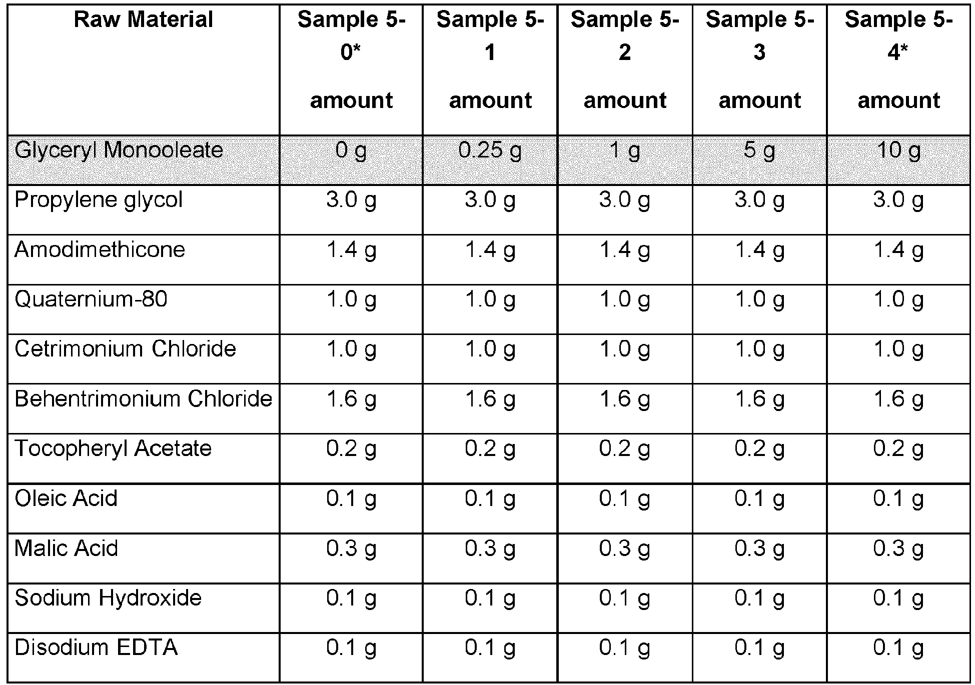

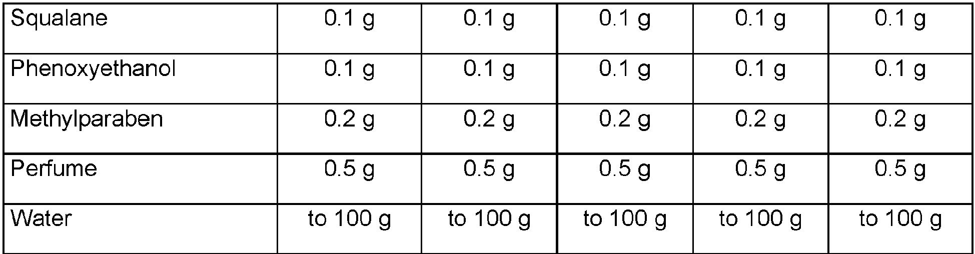

* not within the scope of the presently claimed invention

### Test Methodology - Half Head Tests

The ability of the foregoing leave-on treatment examples to condition the hair was examined by half head comparisons on humans/panelists. The hair of the panelists was parted in the middle into two sections from brow to neck. One of the sections was treated with the reference sample 5-0, the other section is treated with one of the 4 test samples 5-1 to 5-4, so overall 4 half head test comparisons per panelist.

This examination was termed half-head test respectively half-head comparison because the test and reference samples were applied to one half of the head of a panelist respectively, thus enabling a direct comparison under absolutely identical test conditions (identical hair structure, degree of hair damage, hair color etc.).

The following hair conditioning criteria were judged by experienced, professional stylists: wet and dry combability, wet feel, anti-frizz, hair shine and dry feel.

### Evaluation Criteria for Half Head Examination:

### Wet and Dry Combability:

Combability of the hair was assessed by placing an aluminum comb parallel to the middle parting and running it through the hair to the shoulder. The comb must remain at a 90° angle throughout and remain in contact with the scalp throughout combing to avoid varying comb angles. The amount of resistance/effort needed during combing was the basis for evaluating the product as easier to comb/more difficult to comb. This test can be carried out on wet hair and on dry hair as well. The evaluation was conducted manually and was compared to the combability of the hair with reference conditioner.

### Wet feel:

The wet feel of the hair was evaluated by taking the hair between the thumb and first two fingers with light pressure, moving from the hair roots to the ends, or by spreading the fingers and sliding them through the hair from roots to ends. If the hair slides through the fingers without any problem, we refer to a smooth feel; if the hair does not slide smoothly through the fingers, we refer to a rough feel.

### Anti-Frizz:

Hair Frizz was evaluated optically - on straight hair styles via assessing if short strands sticking up at the part and throughout the hair length that "project away from the main body of hair", and on curly hair via assessing how much strands of wavy or curly hair do not align with others to form a defined wave or curl. The less hair sticking up on straight hair and the more defined/less unruly curls or waves are observed, the less frizzy the hair was.

### Dry Feel:

When the hair was completely dry, the evaluating stylist assessed the dry feel of the hair. This was assessed by running the hair from root to end between the thumb and middle and index fingers, while simultaneously applying light pressure; or alternatively, by running lightly outspread fingers through the hair from root to end. If the hair runs easily through the fingers, this is referred to as a smooth feel; if the hair is impeded from running easily through the fingers, this is referred to as a coarse feel.

### Hair Shine:

Hair shine was evaluated by looking at the reflection of light on the hair under standard conditions (natural daylight or a daylight lamp). From a distance of 0.5 m, and with slight head movements by the model, the light reflection or shine was evaluated as more/less.

Half head examination was carried out with 5 test persons, the numbers indicated how many test persons/panelists were judged for each criterion.

### Test sample 5-1 (0.25% GMO), comparison vs. reference (0% GMO):

| Criterion | Test sample 5-1 better than reference sample 5-0 | Test sample 5-1 equal to reference sample 5-0 | Test sample 5-1 worse than reference sample 5-0 |
|---|---|---|---|
| Wet Combability | **3** | **2** | **0** |
| Wet Feel | **5** | **0** | **0** |
| Dry Combability* | **4** | **0** | **0** |
| Anti-Frizz | **5** | **0** | **0** |
| Dry Feel | **5** | **0** | **0** |
| Shine | **5** | **0** | **0** |

| | | | |
|---|---|---|---|
| * Dry combability half head tests were carried out among four test persons/panelists. | | | |

### Test sample 5-2 (1% GMO), comparison vs. reference (0% GMO):

| Criterion | Test sample 5-2 better than reference sample 5-0 | Test sample 5-2 equal toreference sample 5-0 | Test sample 5-2 worse than reference sample 5-0 |
|---|---|---|---|
| Wet Combability | **4** | **1** | **0** |
| Wet Feel | **5** | **0** | **0** |
| Dry Combability* | **3** | **1** | **0** |
| Anti-Frizz | **4** | **1** | **0** |
| Dry Feel | **5** | **0** | **0** |
| Shine | **5** | **0** | **0** |

| | | | |
|---|---|---|---|
| * Dry combability half head tests were carried out among four test persons/panelists. | | | |

### Test sample 5-3 (5% GMO), comparison vs. reference (0% GMO):

| Criterion | Test sample 5-3 better than reference sample 5-0 | Test sample 5-3 equal to reference sample 5-0 | Test sample 5-3 worse than reference sample 5-0 |
|---|---|---|---|
| Wet Combability | **4** | **1** | **0** |
| Wet Feel | **5** | **0** | **0** |
| Dry Combability** | **1** | **0** | **2** |
| Anti-Frizz | **4** | **0** | **1** |
| Dry Feel | **5** | **0** | **0** |
| Shine | **5** | **0** | **0** |

| | | | |
|---|---|---|---|
| ** Dry combability half head tests were carried out among three test persons/panelists. | | | |

### Test sample 5-4 (10% GMO), comparison vs. reference (0% GMO):

| Criterion | Test sample 5-4 better than reference sample 5-0 | Test sample 5-4 equal to reference sample 5-0 | Test sample 5-4 worse than reference sample 5-0 |
|---|---|---|---|
| Wet Combability*** | **3** | **0** | **0** |
| Wet Feel*** | **3** | **0** | **0** |
| Dry Combability*** | **3** | **1** | **0** |
| Anti-Frizz*** | **4** | **1** | **0** |
| Dry Feel*** | **5** | **0** | **0** |
| Shine*** | **5** | **0** | **0** |

| | | | |
|---|---|---|---|
| *** Half head test were carried out among three test persons/panelists only, because product resulted in too much heaviness/loading and needed to be stopped on two out of five panelists. | | | |

The results of the half head examinations indicate clearly that glyceryl monooleate contributed to the excellent hair conditioning composition. GMO does improve the conditioning performance up to a concentration of max. 5 wt.%, as of this concentration GMO does result in too much heaviness/loading of the hair which is unacceptable for the majority of consumers.

## Claims

1. A hair treatment composition comprising:
a) at least one C3-C13 saturated or unsaturated dicarboxylic acid which is unsubstituted or substituted with 1, 2, 3 or 4 hydroxyl groups at a weight percentage concentration of from 0.05 wt.% to 15.0 wt.%;
b) at least one C10-C30 saturated or unsaturated monocarboxylic acid at a weight percentage concentration of 0.05 wt.% to 1.0 wt.%;
c) at least one C10-C50 saturated or unsaturated hydrocarbon at a weight percentage concentration of 0.01 wt.% to 10.0 wt.%;
d) at least one fatty acid ester of a C10-C30 saturated or unsaturated monocarboxylic acid and a C3-C6 mono-ol, diol or triol at a weight percentage concentration of 0.1 wt.% to 5.0 wt.%;
e) at least one cosmetically acceptable aqueous or aqueous-organic medium;
wherein the components a) and b) are present in the form of a free acid or a cosmetically acceptable salt thereof and
all weight percentages are relative to the total weight of the composition.

2. The hair treatment composition according to claim 1, wherein the component a) is at least one C3-C6 saturated or unsaturated dicarboxylic acid which is unsubstituted or substituted with 1, 2, 3 or 4 hydroxyl groups.

3. The hair treatment composition according to claim 2, wherein the at least one C3-C6 saturated or unsaturated dicarboxylic acid which is unsubstituted or substituted with 1, 2, 3 or 4 hydroxyl groups is selected from the group consisting of malonic acid, succinic acid, glutaric acid, adipic acid, malic acid, 2-methyl malic acid, hydroxyglutaric acid, fumaric acid, maleic acid, itaconic acid, glutaconic acid, tartaric acid, 2-methyltartaric acid, 2-hydroxy-3-methylglutaric acid, hydroxyadipic acid and hydroxyglutaric acid.

4. The hair treatment composition according to claim 2, wherein the component a) is at least one C3-C6 saturated dicarboxylic acid which is substituted with 1, 2, 3 or 4 hydroxyl groups.

5. The hair treatment composition according to claim 4, wherein the at least one C3-C6 saturated dicarboxylic acid which is substituted with 1, 2, 3 or 4 hydroxyl groups is selected from the group consisting of malic acid, 2-methyl malic acid, hydroxyglutaric acid, tartaric acid, 2-methyltartaric acid, 2-hydroxy-3-methylglutaric acid, hydroxyadipic acid, and hydroxyglutaric acid.

6. The hair treatment composition according to any of the preceding claims, wherein the component b) is at least one C16-C22 saturated or unsaturated monocarboxylic acid.

7. The hair treatment composition according to any of the preceding claims, wherein the component c) is selected from the group consisting of paraffins and saturated acyclic terpanes.

8. The hair treatment composition according to claim 7, wherein the saturated acyclic terpanes are selected from the group consisting of squalane, isosqualane and neosqualane.

9. The hair treatment composition according to any of the preceding claims, wherein the component d) is at least one fatty acid ester of a C16-C22 saturated or unsaturated monocarboxylic acid and a C3-C6 triol.

10. The hair treatment composition according to any of the preceding claims, wherein the component a) is present at a weight percentage concentration in the range of from 0.1 wt.% to 10.0 wt.%.

11. The hair treatment composition according to any of the preceding claims, wherein the component c) is present at a weight percentage concentration in the range of 0.02 wt.% to 4.0 wt.%.

12. The hair treatment composition according to any of the preceding claims, wherein the component d) is present at a weight percentage concentration in the range of 0.15 wt.% to 3.0 wt.%.

13. The hair treatment composition according to any of the preceding claims, wherein the ratio of the weight percentage concentration of the component a) to the weight percentage concentration of the component b) is in the range of 10.0:1.0 to 1.0:1.0, preferably in the range of 5.0:1.0 to 2.0:1.0.

14. The hair treatment composition according to any of the preceding claims, wherein the hair treatment composition is selected from the group consisting of hair conditioners, hair creams, hair shampoos, dry shampoos, hair rinses, hair liquids, hair oils, hair pomades, hair foams, hair sprays, hair tonics and hair lotions.

15. A method for treating hair comprising at least the step of
A) applying to the hair a hair treatment composition according to any of claims 1 to 14.

16. The use of the hair treatment composition according to any of claims 1 to 14 for repairing damaged hair.

## Patentansprüche

1. Haarbehandlungszusammensetzung, aufweisend:
a) mindestens eine gesättigte oder ungesättigte C3-C13-Dicarbonsäure, welche unsubstituiert oder mit 1, 2, 3 oder 4 Hydroxylgruppen substituiert ist, in einer gewichtsprozentualen Konzentration von 0,05 Gew.-% bis 15,0 Gew.-%;
b) mindestens eine gesättigte oder ungesättigte C10-C30-Monocarbonsäure in einer gewichtsprozentualen Konzentration von 0,05 Gew.-% bis 1,0 Gew.-%;
c) mindestens einen gesättigten oder ungesättigten C10-C50-Kohlenwasserstoff in einer gewichtsprozentualen Konzentration von 0,01 Gew.-% bis 10,0 Gew.-%;
d) mindestens einen Fettsäureester einer gesättigten oder ungesättigten C10-C30-Monocarbonsäure und eines C3-C6-Monools, -diols oder -triols in einer gewichtsprozentualen Konzentration von 0,1 Gew.-% bis 5,0 Gew.-%;
e) mindestens ein kosmetisch akzeptables wässriges oder wässrig-organisches Medium;
wobei die Komponenten a) und b) in Form einer freien Säure oder eines kosmetisch akzeptablen Salzes davon vorliegen; und
alle Gewichtsprozente sich auf das Gesamtgewicht der Zusammensetzung beziehen.

2. Haarbehandlungszusammensetzung nach Anspruch 1, wobei die Komponente a) mindestens eine gesättigte oder ungesättigte C3-C6-Dicarbonsäure ist, welche unsubstituiert oder mit 1, 2, 3 oder 4 Hydroxylgruppen substituiert ist.

3. Haarbehandlungszusammensetzung nach Anspruch 2, wobei die mindestens eine gesättigte oder ungesättigte C3-C6-Dicarbonsäure, welche unsubstituiert oder mit 1, 2, 3 oder 4 Hydroxylgruppen substituiert ist, ausgewählt ist aus der Gruppe bestehend aus Malonsäure, Bernsteinsäure, Glutarsäure, Adipinsäure, Äpfelsäure, 2-Methyl-Äpfelsäure, Hydroxyglutarsäure, Fumarsäure, Maleinsäure, Itaconsäure, Glutaconsäure, Glutarsäure, Adipinsäure, Äpfelsäure, 2-Methyl-Äpfelsäure, Hydroxyglutarsäure, Fumarsäure, Maleinsäure, Itaconsäure, Glutaconsäure, Weinsäure, 2-Methylweinsäure, 2-Hydroxy-3-methylglutarsäure, Hydroxyadipinsäure und Hydroxyglutarsäure.

4. Haarbehandlungszusammensetzung nach Anspruch 2, wobei die Komponente a) mindestens eine gesättigte C3-C6-Dicarbonsäure ist, welche mit 1, 2, 3 oder 4 Hydroxylgruppen substituiert ist.

5. Haarbehandlungszusammensetzung nach Anspruch 4, wobei die mindestens eine gesättigte C3-C6-Dicarbonsäure, welche mit 1, 2, 3 oder 4 Hydroxylgruppen substituiert ist, aus der Gruppe bestehend aus Apfelsäure, 2-Methyl-Äpfelsäure, Hydroxyglutarsäure, Weinsäure, 2-Methylweinsäure, 2-Hydroxy-3-methylglutarsäure, Hydroxyadipinsäure und Hydroxyglutarsäure ausgewählt ist.

6. Haarbehandlungszusammensetzung nach einem der vorhergehenden Ansprüche, wobei die Komponente b) mindestens eine gesättigte oder ungesättigte C16-C22-Monocarbonsäure ist.

7. Haarbehandlungszusammensetzung nach einem der vorhergehenden Ansprüche, wobei die Komponente c) ausgewählt ist aus der Gruppe der Paraffine und der gesättigten azyklischen Terpane.

8. Haarbehandlungszusammensetzung nach Anspruch 7, wobei die gesättigten azyklischen Terpane ausgewählt sind aus der Gruppe bestehend aus Squalan, Isosqualan und Neosqualan.

9. Haarbehandlungszusammensetzung nach einem der vorhergehenden Ansprüche, wobei die Komponente d) mindestens ein Fettsäureester einer gesättigten oder ungesättigten C16-C22-Monocarbonsäure und eines C3-C6-Triols ist.

10. Haarbehandlungszusammensetzung nach einem der vorhergehenden Ansprüche, wobei die Komponente a) in einer gewichtsprozentualen Konzentration im Bereich von 0,1 Gew.-% bis 10,0 Gew.-% vorhanden ist.

11. Haarbehandlungszusammensetzung nach einem der vorhergehenden Ansprüche, wobei die Komponente c) in einer gewichtsprozentualen Konzentration im Bereich von 0,02 Gew.-% bis 4,0 Gew.-% vorhanden ist.

12. Haarbehandlungszusammensetzung nach einem der vorhergehenden Ansprüche, wobei die Komponente d) in einer gewichtsprozentualen Konzentration im Bereich von 0,15 Gew.-% bis 3,0 Gew.-% vorhanden ist.

13. Haarbehandlungszusammensetzung nach einem der vorhergehenden Ansprüche, wobei das Verhältnis der gewichtsprozentualen Konzentration der Komponente a) zur gewichtsprozentualen Konzentration der Komponente b) im Bereich von 10,0:1,0 bis 1,0:1,0, vorzugsweise im Bereich von 5,0:1,0 bis 2,0:1,0, liegt.

14. Haarbehandlungszusammensetzung nach einem der vorhergehenden Ansprüche, wobei die Haarbehandlungszusammensetzung ausgewählt ist aus der Gruppe bestehend aus Haarkuren, Haarcremes, Haarshampoos, Trockenshampoos, Haarspülungen, Haarflüssigkeiten, Haarölen, Haarpomaden, Haarschäumen, Haarsprays, Haartonika und Haarlotionen.

15. Verfahren zur Haarbehandlung, welches mindestens den Schritt umfasst A) Auftragen einer Haarbehandlungszusammensetzung nach einem der Ansprüche 1 bis 14 auf das Haar.

16. Verwendung der Haarbehandlungszusammensetzung nach einem der Ansprüche 1 bis 14 zur Wiederherstellung von geschädigtem Haar.

## Revendications

1. Composition de traitement capillaire comprenant :
a) au moins un acide dicarboxylique saturé ou insaturé en C3 à C13 qui est non substitué ou substitué par 1, 2, 3 ou 4 groupes hydroxyle à une concentration en pourcentage en poids de 0,05 % en poids à 15,0 % en poids ;
b) au moins un acide monocarboxylique saturé ou insaturé en C10 à C30 à une concentration en pourcentage en poids de 0,05 % en poids à 1,0 % en poids ;
c) au moins un hydrocarbure saturé ou insaturé en C10 à C50 à une concentration en pourcentage en poids de 0,01 % en poids à 10,0 % en poids ;
d) au moins un ester d'acide gras d'un acide monocarboxylique saturé ou insaturé en C10 à C30 et d'un monool, diol ou triol en C3 à C6 à une concentration en pourcentage pondéral de 0,1 % en poids à 5,0 % en poids ;
e) au moins un milieu aqueux ou aqueux-organique cosmétiquement acceptable ;
dans laquelle les composants a) et b) sont présents sous la forme d'un acide libre ou d'un sel cosmétiquement acceptable de celui-ci et
tous les pourcentages en poids sont relatifs au poids total de la composition.

2. Composition de traitement capillaire selon la revendication 1, dans laquelle le composant a) est au moins un acide dicarboxylique saturé ou insaturé en C3 à C6 qui est non substitué ou substitué par 1, 2, 3 ou 4 groupes hydroxyle.

3. Composition de traitement capillaire selon la revendication 2, dans laquelle l'au moins un acide dicarboxylique saturé ou insaturé en C3 à C6 qui est non substitué ou substitué par 1, 2, 3 ou 4 groupes hydroxyle est choisi dans le groupe constitué par l'acide malonique, l'acide succinique, l'acide glutarique, l'acide adipique, l'acide malique, l'acide 2-méthylmalique, l'acide hydroxyglutarique, l'acide fumarique, l'acide maléique, l'acide itaconique, l'acide glutaconique, l'acide tartrique, l'acide 2-méthyltartrique, l'acide 2-hydroxy-3-méthylglutarique, l'acide hydroxyadipique et l'acide hydroxyglutarique.

4. Composition de traitement capillaire selon la revendication 2, dans laquelle le composant a) est au moins un acide dicarboxylique saturé en C3 à C6 qui est substitué par 1, 2, 3 ou 4 groupes hydroxyle.

5. Composition de traitement capillaire selon la revendication 4, dans laquelle l'au moins un acide dicarboxylique saturé C3 à C6 qui est substitué par 1, 2, 3 ou 4 groupes hydroxyle est choisi dans le groupe constitué par l'acide malique, l'acide 2-méthylmalique, l'acide hydroxyglutarique, l'acide tartrique, l'acide 2-méthyltartrique, l'acide 2-hydroxy-3-méthylglutarique, l'acide hydroxyadipique et l'acide hydroxyglutarique.

6. Composition de traitement capillaire selon l'une quelconque des revendications précédentes, dans laquelle le composant b) est au moins un acide monocarboxylique saturé ou insaturé en C16 à C22.

7. Composition de traitement capillaire selon l'une quelconque des revendications précédentes, dans laquelle le composant c) est choisi dans le groupe constitué par les paraffines et les terpanes acycliques saturés.

8. Composition de traitement capillaire selon la revendication 7, dans laquelle les terpanes acycliques saturés sont choisis dans le groupe constitué par le squalane, l'isosqualane et le néosqualane.

9. Composition de traitement capillaire selon l'une quelconque des revendications précédentes, dans laquelle le composant d) est au moins un ester d'acide gras d'un acide monocarboxylique saturé ou insaturé en C16 à C22 et d'un triol en C3 à C6.

10. Composition de traitement capillaire selon l'une quelconque des revendications précédentes, dans laquelle le composant a) est présent à une concentration en pourcentage en poids dans la plage de 0,1 % en poids à 10,0 % en poids.

11. Composition de traitement capillaire selon l'une quelconque des revendications précédentes, dans laquelle le composant c) est présent à une concentration en pourcentage en poids dans la plage de 0,02 % en poids à 4,0 % en poids.

12. Composition de traitement capillaire selon l'une quelconque des revendications précédentes, dans laquelle le composant d) est présent à une concentration en pourcentage en poids dans la plage de 0,15 % en poids à 3,0 % en poids.

13. Composition de traitement capillaire selon l'une quelconque des revendications précédentes, dans laquelle le rapport de la concentration en pourcentage en poids du composant a) à la concentration en pourcentage en poids du composant b) est dans la plage de 10,0:1,0 à 1,0:1,0, de préférence dans la plage de 5,0:1,0 à 2,0:1,0.

14. Composition de traitement capillaire selon l'une quelconque des revendications précédentes, dans laquelle la composition de traitement capillaire est choisie dans le groupe constitué par les après-shampooings capillaires, les crèmes capillaires, les shampooings capillaires, les shampooings secs, les produits de rinçage capillaire, les liquides capillaires, les huiles capillaires, les pommades capillaires, les mousses capillaires, les laques capillaires, les toniques capillaires et les lotions capillaires.

15. Méthode de traitement capillaire comprenant au moins l'étape consistant à
A) appliquer sur les cheveux une composition de traitement capillaire selon l'une quelconque des revendications 1 à 14.

16. Utilisation de la composition de traitement capillaire selon l'une quelconque des revendications 1 à 14 pour réparer les cheveux abîmés.
